# EUROPÄISCHE PATENTSCHRIFT

(11) **EP 1 169 012 B1**
(45) Veröffentlichungstag und Bekanntmachung des Hinweises auf die Patenterteilung: **06.09.2006**
(21) Anmeldenummer: 00920626.9
(22) Anmeldetag: 30.03.2000
(51) Int. Cl.: A61K 8/41, A61Q 5/10

(54) **4-AMINO-2-AMINOMETHYL-PHENOL-DERIVATE ENTHALTENDE FÄRBEMITTEL UND DEREN VERWENDUNG ZUM FÄRBEN VON KERATINFASERN**
COLORANTS CONTAINING 4-AMINO-2-AMINOMETHYL-PHENOL DERIVATIVES AND THEIR USE FOR COLORING KERATIN FIBERS
COLORANTS CONTENANT DES DERIVES DE 4-AMINO-2-AMINOMETHYLPHENOL ET LEUR UTILISATION POUR COLORER DES FIBRES DE KERATINE

(30) Priorität: 09.04.1999 DE 19916033
(43) Veröffentlichungstag der Anmeldung: 09.01.2002
(73) Patentinhaber: Henkel Kommanditgesellschaft auf Aktien, 40589 Düsseldorf (DE)
(72) Erfinder: HÖFFKES, Horst, D-40595 Düsseldorf (DE); OBERKOBUSCH, Doris, D-40591 Düsseldorf (DE); HITZ, Melanie, D-41542 Dormagen (DE)
(86) Internationale Anmeldenummer: PCT/EP2000/002803
(87) Internationale Veröffentlichungsnummer: WO 2000/061088

(56) Entgegenhaltungen:
- DE-A- 3 543 345
- DE-A- 4 429 343
- DE-C- 19 631 690

## Beschreibung

Die vorliegende Erfindung betrifft Färbemittel, die als Entwicklerkomponente spezielle Derivate des 4-Amino-2-Aminomethylphenols enthalten, sowie die Verwendung dieser Mittel zum Färben von Keratinfasem.

Für das Färben von Keratinfasern, insbesondere menschlichen Haaren, spielen die sogenannten Oxidationsfärbemittel wegen ihrer intensiven Farben und guten Echtheitseigenschaften eine bevorzugte Rolle. Solche Färbemittel enthalten Oxidationsfarbstoffvorprodukte, sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehrerer Kupplerkomponenten die eigentlichen Farbstoffe aus.

Gute Oxidationsfarbstoffvorprodukte sollen in erster Linie folgende Voraussetzungen erfüllen: Sie müssen bei der oxidativen Kupplung die gewünschten Farbnuancen in ausreichender Intensität und Echtheit ausbilden. Sie müssen ferner ein gutes Aufziehvermögen auf die Faser besitzen, wobei insbesondere bei menschlichen Haaren keine merklichen Unterschiede zwischen strapaziertem und frisch nachgewachsenem Haar bestehen dürfen (Egalisiervermögen). Schließlich sollen sie - falls als Haarfärbemittel zur Anwendung kommend - die Kopfhaut nicht zu sehr anfärben, und vor allem sollen sie in toxikologischer und dermatologischer Hinsicht unbedenklich sein.

Als Entwicklerkomponenten werden üblicherweise primäre aromatische Amine mit einer weiteren, in para- oder ortho-Position befindlichen, freien oder substituierten Hydroxy- oder Aminogruppe, Diaminopyridinderivate, heterocyclische Hydrazone, 4-Aminopyrazolderivate sowie 2,4,5,6-Tetraaminopyrimidin und dessen Derivate eingesetzt. Als Kupplerkomponenten werden in der Regel m-Phenylendiaminderivate, Naphthole, Resorcin und Resorcinderivate, Pyrazolone und m-Aminophenole verwendet.

Allein mit einer Entwicklerkomponente oder einer speziellen Kuppler/Entwicklerkombination gelingt es in der Regel nicht, eine auf dem Haar natürlich wirkende Farbnuance zu erhalten. In der Praxis werden daher üblicherweise Kombinationen verschiedener Entwickler- und/oder Kupplerkomponenten eingesetzt. Die durch die Farbstoffkombinationen erzielbaren Färbungen sollen beständig sein gegen Licht, Wärme, Schweiß, Reibung und den Einfluß chemischer Reduktionsmittel, z.B. Dauerwellenflüssigkeiten. Weiterhin soll die erzielte Färbung durch Blondierung leicht wieder aus dem Haar entfernt werden können, falls sie doch nicht den individuellen Wünschen der einzelnen Person entspricht und rückgängig gemacht werden soll. Es besteht daher die Aufgabe, neue Entwickler-Kupplerkombinationen zu entwickeln, mit denen sich ausdrucksvolle Farbtöne über das gesamte für Haarfärbemittel relevante Spektrum von gelb, orange, braun bis schwarz erzielen lassen und die auch in toxikologischer Hinsicht ein Fortschritt gegenüber dem Stand der Technik sind.

Überraschenderweise wurde nun gefunden, daß Entwickler-Kupplerkombinationen, die spezielle 4-Amino-2-aminomethylderivate als Entwicklerkomponente enthalten, die an derartige Kombinationen gestellten Aufgaben in einem hohen Maße erfüllen.

Gegenstand der vorliegenden Erfindung sind daher Mittel zum Färben keratinischer Fasern, die mindestens eine Entwicklerkomponente der Formel (I) 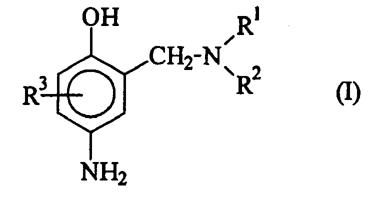 wobei R¹ und R² unabhängig voneinander für eine C₁-bis C₄-Alkylgruppe stehen und R³ für Wasserstoff, ein Halogenatom, eine C₁-bis C₄-Alkylgruppe, eine C₁-bis C₄₋Alkoxygruppe oder eine C₁-bis C₄-Hydroxyalkylgruppe steht und mindestens eine Kupplerkomponente ausgewählt aus einer Gruppe, gebildet von 3-Amino-2-chlor-6-methylphenol, 3-Trifluoroacetylamino-2-chlor-6-methylphenol, 5-Amino-4-chlor-2-methylphenol und 2,4-Dichlor-3-aminophenol, enthalten.

Unter keratinischen Fasern sind erfindungsgemäß Pelze, Wolle, Federn und insbesondere menschliche Haare zu verstehen. Obwohl die erfindungsgemäßen Färbemitteln in erster Linie zum Färben von keratinischen Fasern geeignet sind, steht prinzipiell einer Verwendung auf anderen Gebieten nichts entgegen.

Beispiele für die als Substituenten in den erfindungsgemäßen Verbindungen genannten C₁- bis C₄-Alkylgruppen, sind die Gruppen Methyl, Ethyl, Propyl, Isopropyl und Butyl. Ethyl- und Methylgruppen sind bevorzugte Alkylgruppen. Ethylgruppen sind ganz besonders bevorzugt.

Besonders bevorzugt sind Verbindungen der Formel (I), bei denen der Rest R³ für Wasserstoff steht.

Eine ganz besonders bevorzugte Verbindung der Formel (I) ist das 4-Amino-2-((diethylamino)methyl)phenol. Färbungen, die mit dieser Verbindung in Kombination mit den erfindungsgemäßen Kupplern erhalten werden, zeichnen sich durch eine große Nuancenvielfalt sowie hohe Licht- und Shampoonierechtheit aus.

Der Einsatz von 4-Amino-2-aminomethylderivaten der Formel (I) in Haarfärbemitteln ist bereits in der Literatur beschrieben worden. So beschäftigt sich beispielsweise die DE-A1-35 43 345 allgemein mit Derivaten von 4-Amino-2-aminomethylphenolen. Weiterhin sind in der EP-A1-857 479 Haarfärbemittel beschrieben worden, die 4-Amino-2-((diethylamino)methyl)phenol in Kombination mit 5-Amino-2-methoxyphenol enthalten. Diesen eher vagen Angaben läßt sich jedoch keinerlei Hinweis auf die mit den erfindungsgemäßen Kombinationen erzielbaren Effekte entnehmen.

In einer Ausführungsform der vorliegenden Erfindung können die Färbemittel noch weitere Entwicklerkomponenten enthalten. Erfindungsgemäß bevorzugte Entwicklerkomponenten sind p-Phenylendiamin, p-Toluylendiamin, p-Aminophenol, o-Aminophenol, 1-(2'-Hydroxyethyl)-2,5-diaminobenzol, N,N-Bis-(2-hydroxy-ethyl)-p-phenylendiamin, 2-(2,5-Diaminophenoxy)-ethanol, 1-Phenyl-3-carboxyamido-4-amino-pyrazolon-5, 4-Amino-3-methylphenol, 2,4,5,6-Tetraamino-pyrimidin, 2-Hydroxy-4,5,6-triaminopyrimidin, 4-Hydroxy-2,5,6-triaminopyrimidin, 2,4-Dihydroxy-5,6-diaminopyrimidin, 2-Dimethylamino-4,5,6-triaminopyrimidin, 2-Hydroxyethylaminomethyl-4-amino-phenol, 4,4'-Diaminodiphenylamin, 4-Amino-3-fluorphenol, 2-Aminomethyl-4-amino-phenol, 2-Hydroxymethyl-4-aminophenol, Bis-(2-hydroxy-5-aminophenyl)-methan, 1,4-Bis-(4-aminophenyl)-diazacycloheptan, 1,3-Bis-(N-(2-hydroxyethyl)-N(4-aminophenylamino))-2-propanol, 4-Amino-2-(2-hydroxyethoxy)-phenol, 1,10-Bis-(2,5-diaminophenyl)-1,4,7,10-tetraoxadecan sowie 4,5-Diaminopyrazol-Derivate nach EP 0 740 931 bzw. WO 94/08970 wie z.B. 4,5-Di-amino-1-(2'-hydroxyethyl)-pyrazol.

Besonders bevorzugte Entwicklerkomponenten sind p-Phenylendiamin, p-Toluylendiamin, p-Aminophenol, 1-(2'-Hydroxyethyl)-2,5-diaminobenzol, 4-Amino-3-methylphenol, 2,4,5,6-Tetraaminopyrimidin, 2-Hydroxy-4,5,6-triaminopyrimidin, 4-Hydroxy-2,5,6-triaminopyrimidin, 4,5-Diamino-1-(2-hydroxyethyl)-pyrazol, 2-Aminomethyl-4-aminophenol, N,N-Bis-(2-hydroxyethyl)-p-phenylendiamin, Bis-(2-hydroxy-5-aminophenyl)-methan, 1,10-Bis-(2,5-diaminophenyl)-1,4,7,10-tetraoxadecan und o-Aminophenol.

Zur Nuancierung der erzielbaren Farbtöne können die erfindungsgemäßen Mittel weiterhin noch eine oder mehrere weitere Kupplerkomponenten enthalten. Kupplersubstanzen sind häufig aromatische oder heterocyclische Ringsysteme, die zwei reaktive Gruppen in meta-Stellung aufweisen. Als Kupplerkomponenten werden in der Regel m-Phenylendiaminderivate, Naphthole, Resorcin und Resorcinderivate, Pyrazolone und m-Aminophenolderivate verwendet.

Erfindungsgemäß bevorzugte Kupplerkomponenten sind
- m-Aminophenol und dessen Derivate wie beispielsweise 5-Amino-2-methylphenol, 5--(3-Hydroxypropylamino)-2-methylphenol, -2-Hydroxy-4-aminophenoxyethanol, 3-Amino-6-methoxy-2-methylaminophenol, 2,6-Dimethyl-3-aminophenol, 5-Amino-4-methoxy-2-methylphenol, 5-(2`-Hydroxyethyl)-amino-2-methylphenol, 3-(Diethylamino)-phenol, N-Cyclopentyl-3-aminophenol, 1,3-Dihydroxy-5-(methylamino)-benzol und 3-(Ethylamino)-4-methylphenol,
- o-Aminophenol und de Derivate,
- m-Diaminobenzol und dessen Derivate wie beispielsweise 1-Methoxy-2-amino-4-(2'-hydroxyethylamino)benzol, 1,3-Bis-(2,4-diaminophenyl)-propan und 1-Amino-3-bis-(2`-hydroxyethyl)-aminobenzol,
- o-Diaminobenzol und dessen Derivate wie beispielsweise 2,3-Diamino-1-methylbenzol,
- Di- beziehungsweise Trihydroxybenzolderivate wie beispielsweise Resorcin, Resorcinmonomethylether, 2-Methylresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin und 2,3-Diamino-6-methoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,6-Dihydroxynaphthalin, 1,8-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate,
- Pyrazolderivate,
- Indolderivate wie beispielsweise 7-Hydroxyindol,
- Methylendioxybenzolderivate wie beispielsweise 3,4-Methylendioxyphenol, 1-Hydroxy-3,4-methylendioxybenzol, 1-Amino-3,4-methylendioxybenzol und 1-(2'-Hydroxyethyl)-amino-3,4-methylendioxybenzol.

Besonders bevorzugte weitere Kupplerkomponenten im Sinne der Erfindung sind 1-Naphthol, 5-Amino-2-methylphenol, 2-Amino-3-hydroxypyridin, 3-Amino-2-methylamino-6-methoxypyridin, Resorcin, 4-Chlorresorcin, 2-Methylresorcin, m-Aminophenol und o-Aminophenol.

Die Entwickler- und Kupplerkomponenten werden üblicherweise in freier Form eingesetzt. Bei Substanzen mit Aminogruppen kann es aber bevorzugt sein, sie in Salzform, insbesondere in Form der Hydrochloride und Sulfate, einzusetzen.

Die erfindungsgemäßen Haarfärbemittel enthalten sowohl die Entwicklerkomponenten als auch die Kupplerkomponenten bevorzugt in einer Menge von 0,005 bis 20 Gew.-%, vorzugsweise 0,1 bis 5 Gew.-%, jeweils bezogen auf das gesamte Oxidationsfärbemittel. Dabei werden Entwicklerkomponenten und Kupplerkomponenten im allgemeinen in etwa molaren Mengen zueinander eingesetzt. Wenn sich auch der molare Einsatz als zweckmäßig erwiesen hat, so ist ein gewisser Überschuß einzelner Oxidationsfarbstoffvorprodukte nicht nachteilig, so daß Entwicklerkomponenten und Kupplerkomponenten in einem Mol-Verhältnis von 1:0,5 bis 1:3, insbesondere 1:1 bis 1:2, enthalten sein können.

In einer bevorzugten Ausführungsform enthalten die erfindungsgemäßen Haarfärbemittel zur weiteren Modifizierung der Farbnuancen neben den Oxidationsfarbstoffvorprodukten zusätzlich übliche direktziehende Farbstoffe. Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, Basic Yellow 57, Disperse Orange 3, HC Red 3, HC Red BN, Basic Red 76, HC Blue 2, HC Blue 12, Disperse Blue 3, Basic Blue 99, HC Violet 1, Disperse Violet 1, Disperse Violet 4, Disperse Black 9, Basic Brown 16 und Basic Brown 17 bekannten Verbindungen sowie 4-Amino-2-nitrodiphenylamin-2'-carbonsäure, 6-Nitro-1,2,3,4-tetrahydrochinoxalin, Hydroxyethyl-2-nitro-toluidin, Pikraminsäure, 2-Amino-6-chloro-4-nitrophenol 4-Ethylamino-3-nitrobenzoesäure und 2-Chloro-6-ethylamino-1-hydroxy-4-nitrobenzol.

Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie beispielsweise Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten.

Weitere in den erfindungsgemäßen Färbemitteln enthaltene Farbstoffkomponenten können auch Indole und Indoline, sowie deren physiologisch verträgliche Salze, sein. Bevorzugte Beispiele sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Aminoindol und 4-Aminoindol. Weiterhin bevorzugt sind 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure, 6-Hydroxyindolin, 6-Aminoindolin und 4-Aminoindolin.

Es ist nicht erforderlich, daß die Oxidationsfarbstoffvorprodukte oder die direktziehenden Farbstoffe jeweils einheitliche Verbindungen darstellen. Vielmehr können in den erfindungsgemäßen HaarFärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

In einer weiteren Ausführungsform der vorliegenden Erfindung enthalten die Haarfärbemittel weiterhin wenigstens einen Farbstoff vom Typ der reaktiven Carbonylverbindungen, ausgewählt aus der Gruppe der aromatischen, heteroaromatischen oder ungesättigten Aldehyde oder Ketone, der Dialdehyde oder Diketone oder der Acetale, Halbaminale oder Iminderivate solcher reaktiver Carbonylverbindungen.

Haarfarbstoffe vom Typ der reaktiven Carbonylverbindungen sind seit längerem bekannt. Geeignete Verbindungen vom Typ der aromatischen Aldehyde sind z.B. in den deutschen Offenlegungsschriften DE 196 30 274 A1 und DE 196 30 275 A1 beschrieben. Geeignete Verbindungen sind z.B. der 2-Hydroxybenzaldehyd, der 4-Hydroxy-3-methoxy-benzaldehyd (Vanillin) und der 4-Hydroxy-3-methoxy-cinnamaldehyd (Coniferylaldehyd).

Geeignete Verbindungen vom Typ der heteroaromatischen Aldehyde sind z.B. in der deutschen Offenlegungsschrift DE 197 17 280 A1 beschrieben. Besonders gut geeignete Farbstoffe sind z.B. trans-β-(2-Furyl)-acrolein, 1-Methylindol-3-aldehyd, 2-(1,3,3-Trimethyl-2-indolinyliden)-acetaldehyd oder Antipyrin-4-aldehyd. Spezielle Produkte dieses Typs mit einer Pyridinium-Gruppe sind in der deutschen Patentanmeldung DE 197 45 356.2 beschrieben, z.B. die sehr gut geeigneten 4-Formyl-1-methylpyridiniumbenzolsulfonat und 4-Formyl-1-methylchinolinium-methan-sulfonat bzw. -methylsulfat.

Geeignete Farbstoffe vom Typ der ungesättigten Aldehyde sind z.B. in der deutschen Offenlegungsschrift DE 197 17 224 A1 beschrieben. Für die vorliegende Erfindung eignet sich besonders gut der Glutaconaldehyd in Form seiner Salze, z.B. seines Alkali- oder Tetrabutylammoniumsalzes oder der 2-Chlor-3-hydroxymethylen-1-cyclohexen-1-aldehyd.

Dialdehyde und Diketone und deren Derivate, die sich als Farbstoffe erfindungsgemäß eignen, sind z.B. alicyclische und cyclische 1,2- und 1,3-Dicarbonyl-verbindungen, wie Isatin, Ninhydrin, Alloxan, Isobarbitursäure, p- und o-Chinone, 1,3-Indandione und deren Derivate. Solche Farbstoffe fmden sich z.B. in der deutschen Offenlegungsschrift DE 43 35 627 A1. Geeignete Verbindungen sind z.B. der Malon-dialdehyd, bevorzugt in Form seines Dimethylacetals, das 2-Nitro-1,3-indandion oder das 2-Acetyl-1,3-cyclohexandion.

Zu den erfindungsgemäß geeigneten Diketonen gehören auch cyclische Dicarbonylverbindungen wie z.B. das Isatin und dessen Derivate, wie sie z.B. in der deutschen Offenlegungsschrift DE 44 09 143 A1 beschrieben sind. Erfindungsgemäß bevorzugt sind z.B. das Isatinsäure-Kaliumsalz, das Isatin-5-Sulfonsäure-Kaliumsalz, das N-Allylisatin, das 1-Piperidinomethylisatin, das 1-Hydroxymethylisatin und das 1-Diethylaminomethylisatin.

Eine weitere geeignete cyclische Dicarbonylverbindung ist z.B. auch die Dehydroascorbinsäure, deren Eignung als Haarfarbstoff aus der deutschen Patentanmeldung DE 197 45 354.6 bekannt ist. Schließlich eignen sich auch die Acetale, Iminderivate und Halbaminale der genannten reaktiven Carbonylverbindungen. Solche Verbindungen werden durch Reaktion der Carboxylgruppe mit primären Alkoholen oder Aminen und ggf. Wasserabspaltung erhalten.

Ausgehend von den ungesättigten Dialdehyden und Diketonen gelangt man dabei in die Gruppe der Merocyanin- und Azomethin-Farbstoffe. Geeignete Imin-Derivate des Glutacondialdehyds sind z.B. das Mono-N-methylanilin-Derivat des Glutaconaldehyds (5-N-Methylanilinopentadienal) oder das N-(5-Anilino-2,4-pentadien-1-yliden)-aniliniumchlorid. Ein weiterer geeigneter vinyloger Cyaninfarbstoff ist das 7-Dimethylamino-2,4,6-heptatrienyliden-dimethylammonium-perchlorat. Solche Verbindungen sind als Haarfärbemittel-Komponenten z.B. aus der deutschen Offenlegungsschrift DE 197 17 223 A1 bekannt.

Viele der genannten reaktiven Carbonylverbindungen färben keratinhaltige Fasern unter Ausprägung verschiedenster Farbnuancen besonders intensiv erst in Kombination mit einer oder mehreren farbverstärkenden Verbindungen mit primärer oder sekundärer Amino- oder Hydroxygruppe, ausgewählt aus der Gruppe der Aminosäuren und Peptide, der aromatischen Amine, Phenole, Aminophenole und stickstoffhaltigen Heterocyclen.

Dabei werden in vielen Fällen auch tiefere (dunklere) Nuancen erhalten.

Geeignete Aminosäuren sind z.B. die natürlich vorkommenden und synthetischen Aminosäuren, z.B. Arginin, Histidin, Phenylalanin, Dihydroxyphenylalanin, Ornithin, Lysin. Geeignete Peptide sind vor allem Oligo- und Polypeptide, die eine ausreichende Wasserlöslichkeit in den erfindungsgemäßen Zubereitungen zur Keratinreduktion aufweisen. Als Beispiele sind z.B. Glutathion oder die in den Hydrolysaten von Kollagen, Keratin, Elastin, Casein, Pflanzenproteinen wie Sojaprotein, Weizengluten oder Mandelprotein enthaltenen Oligopeptide zu nennen.

Geeignete aromatische Amine und Aminophenole sind N,N-Dimethyl-, N,N-Diethyl-, N-(2-Hydroxyethyl)-N-ethyl-, N,N-Bis-(2-hydroxyethyl)-, N-(2-Methoxyethyl-), 2-Chlor-, 2,3-, 2,4- und 2,5-Dichlor-p-phenylendiamin, 2,5-Dihydroxy-4-morpholinoanilin-dihydrobromid, 2-, 3- und 4-Aminophenol, 2-Aminomethyl-4-aminophenol, 2-Hydroxymethyl-4-aminophenol, o-und p-Phenylendiamin, o- und m- Toluylendiamin, 2,5-Diamino-phenol, toluol und phenethol, 4-Amino-3-methylphenol, 2-(2,5-Diaminophenyl)-ethanol, 2,4-Diaminophenoxy-ethanol, 2-(2,5-Diaminophenoxy)-ethanol, 4-Methylamino-, 3-Amino-4-(2'hydroxyethyloxy)-, 3,4-Methylendiamino- und 3,4-Methylendioxyanilin, 3-Amino-2,4-dichlor-, 4-Methylamino-, 2-Methyl-5-amino-, 3-Methyl-4-amino-, 2-Methyl-5-(2-hydroxyethylamino)-, 6-Methyl-3-amino-2-chlor-, 2-Methyl-5-amino-4-chlor-, 3,4-Methylendioxy-, 5-(2-Hydroxyethylamino)-4-methoxy-2-methyl- und 4-Amino-2-hydroxymethylphenol, 1,3-Diamino-2,4-dimethoxybenzol, 2-, 3-, 4-Aminobenzoesäure, -phenylessigsäure, 2,3-, 2,4-, 2,5-, 3,4-, 3,5-Diaminobenzoesäure, 4-, 5-Aminosalicylsäure, 3-Amino-4-hydroxy-, 4-Amino-3-hydroxy-benzoesäure, 2-, 3-, 4-Aminobenzolsulfonsäure, 3-Amino-4-hydroxybenzolsulfonsäure, 4-Amino-3-hydroxynaphthalin-1-sulfonsäure, 6-Amino-7-hydroxynaphthalin-2-sulfonsäure, 7-Amino-4-hydroxynaphthalin-2-sulfonsäure, 4-Amino-5-hydroxynaphthalin-2,7-disulfonsäure, 3-Amino-2-naphthoesäure, 3-Aminophthalsäure, 5-Aminoisophthalsäure, 1,3,5-, 1,2,4-Triaminobenzol, 1,2,4,5-Tetraaminobenzol, 2,4,5-Triaminophenol, Pentaaminobenzol, Hexaaminobenzol, 2,4,6-Triaminoresorcin, 4,5-Diaminobrenzcatechin, 4,6-Diaminopyrogallol, 3,5-Diamino-4-hydroxybrenzcatechin, aromatische Aniline bzw. Phenole mit einem weiteren aromatischen Rest, wie sie in der Formel (II) dargestellt sind 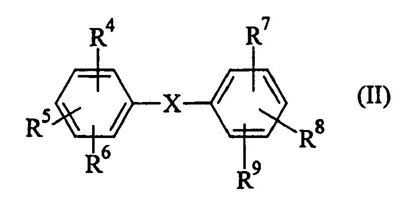 in der R⁴ für eine Hydroxy- oder eine Aminogruppe, die durch C₁₋₄-Alkyl, C₁₋₄₋Hydroxyalkyl- oder C₁₋₄-Alkoxy-C₁₋₄-alkyl substituiert sein kann, steht, R⁵, R⁶, R⁷, R⁸ und R⁹ für Wasserstoff, eine Hydroxy- oder eine Aminogruppe, die durch C₁₋₄-Alkyl-, C₁₋₄-Hydroxyalkyl, C₁₋₄-Aminoalkyl- oder C₁₋₄-Alkoxy-C₁₋₄-alkylgruppe substituiert sein kann, für eine Carbon- oder Sulfonsäuregruppe stehen, und X für eine direkte Bindung, eine gesättigte oder ungesättigte, ggf. durch Hydroxygruppen substituierte Kohlenstoffkette mit 1 bis 4 Kohlenstoffatomen, eine Carbonyl-, Sulfonyl- oder Iminogruppe, ein Sauerstoff- oder Schwefelatom, oder eine Gruppe mit der Formel (III)

Z-(CH₂-Y-CH₂-Z')ₒ (III),

in der Y eine direkte Bindung, eine CH₂- oder CHOH-Gruppe bedeutet,
Z und Z' unabhängig voneinander ein Sauerstoffatom, eine NR¹⁰-Gruppe, worin R¹⁰ Wasserstoff, eine C₁₋₄-Alkyl- oder Hydroxy-C₁₋₄-alkylgruppe bedeutet, die Gruppe -O-(CH₂)ₚ-NH oder NH-(CH₂)ₚ-O, worin p und p' 2 oder 3 sind, stehen und
o eine Zahl von 1 bis 4 bedeutet,
wie beispielsweise 4,4'-Diaminostilben, 4,4'-Diaminostilben-2,2'-disulfonsäure-mono- oder -di-Na-Salz, 4,4'-Diaminodiphenylmethan, -sulfid, -sulfoxid, -amin, 4,4'-Diaminodiphenylamin-2-sulfonsäure, 4,4'-Diaminobenzophenon, -diphenylether, 3,3',4,4'-Tetraaminodiphenyl, 3,3',4,4'-Tetraamino-benzophenon, 1,3-Bis-(2,4-diaminophenoxy)-propan, 1,8-Bis-(2,5-diaminophenoxy)-3,6-dioxaoctan, 1,3-Bis-(4-aminophenylamino)-propan, -2-propanol, 1,3-Bis-[N-(4-aminophenyl)-2-hydroxyethylamino]-2-propanol, N,N-Bis-[2-(4-aminophenoxy)-ethyl]-methylamin, N-Phenyl-1,4-phenylendiamin.

Die vorgenannten Verbindungen können sowohl in freier Form als auch in Form ihrer physiologisch verträglichen Salze, insbesondere als Salze anorganischer Säuren, wie Salz- oder Schwefelsäure, eingesetzt werden.

Geeignete Phenole sind z.B. das 2-, 3- oder 4-Methoxy-, das 3-Dimethylamino-, 2-(2-Hydroxyethyl)- und das 3,4-Methylendioxy-phenol, das Resorcin und das 2-, 4- und 5-Methylresorcin, das 2- und 4-Chlorresorcin, 2,5-Dimethylresorcin, Brenzkatechin, Hydrochinon, Pyrogallol, Phloroglucin, Hydroxyhydrochinon, die 2,4- oder 3,4-Dihydroxybenzoe- oder -phenylessigsäure, die Gallussäure, die 2,4,6-Trihydroxybenzoesäure oder das 2,4,5-Trihydroxyacetophenon, das 1-Naphthol, das 1,5-, 2,3- und 2,7-Dihydroxynaphthalin, die 6-Dimethylamino-4-hydroxy-2-naphthalinsulfonsäure oder die 3,6-Dihydroxy-2,7-naphthalindisulfonsäure.

Geeignete stickstoffhaltige-heterocyclische Verbindungen sind z.B. 2-, 3-, 4-Amino-, 2-Amino-3-hydroxy-, 2,6-Diamino-, 2,5-Diamino-, 2,3-Diamino-, 2-Dimethylamino-5-amino-, 2-Methylamino-3-amino-6-methoxy-, 2,3-Diamino-6-methoxy-, 2,6-Dimethoxy-3,5-diamino-, 2,4,5-Triamino- und 2,6-Dihydroxy-3,4-dimethylpyridin, 2,4-Dihydroxy-5,6-diamino-, 4,5,6-Triamino-, 4-Hydroxy-2,5,6-triamino-, 2-Hydroxy-4,5,6-triamino-, 2,4,5,6-Tetraamino-,-2-Methylamino-4,5,6-triamino-, 2,4-, 4,5-Diamino- und 2-Amino-4-methoxy-6-methyl-pyrimidin, 3-Amino-, 3-Amino-5-hydroxy- und 3,5-Diaminopyrazol, 2-,3-, 8-Aminochinolin, 4-Amino-chinaldin, 2-, 6-Aminonicotinsäure, 5-Aminoisochinolin, 5-, 6-Aminoindazol, 5- und 7-Amino-benzimidazol und -benzothiazol, 2,5-Dihydroxy-4-morpholinoanilin sowie Indol- und Indolinderivate, wie 4-, 5-, 6- und 7-Aminoindol, 5,6-Dihydroxyindol, 5,6-Dihydroxyindolin und 4-Hydroxyindolin. Die vorgenannten Verbindungen können sowohl in freier Form als auch in Form ihrer physiologisch verträglichen Salze, z. B. als Salze anorganischer Säuren, wie Salz- oder Schwefelsäure, eingesetzt werden.

Diese Färbesysteme können noch weiter verstärkt werden durch geeignete stickstoffhaltige Heterocyclen wie z.B. Piperidin, Piperidin-2-, -3- oder -4-carbonsäure, Pyridin, 2-, 3- oder 4-Hydroxypyridin, Imidazol, 1-Methylimidazol, Histidin, Pyrrolidin, Pyrrolidon, Pyrrolidon-5-carbonsäure, Pyrazol, 1,2,4-Triazol, Piperazin sowie deren physiologisch verträglichen Salze.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe), sowie Kapitel 8, Seiten 264-267; Oxidationsfarbstoffvorprodukte), erschienen als Band 7 der Reihe "Dermatology" (Hrg.: Ch. Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Ein zweiter Gegenstand dieser Erfindung ist die Verwendung der vorgenannten Mittel zum Färben keratinischer Fasern.

Zur Herstellung der erfindungsgemäßen Färbemittel können die Oxidationsfarbstoffvorprodukte in einen geeigneten wasserhaltigen Träger eingearbeitet werden. Solche Träger sind zum Zwecke der Haarfärbung z.B. Cremes, Emulsionen, Gele oder auch tensidhaltige schäumende-Lösungen, z.B. Shampoos, Schaumaerosole oder andere Zubereitungen, die für die Anwendung auf dem Haar geeignet sind.

Die erfindungsgemäßen Färbemittel können weiterhin alle für solche Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammonium-glycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, -N-Alkylaminobuttersäuren, N-Alkyliminodipropionsäuren, N-Hydröxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungeprodukte von Ethylenoxid an Sorbitanfettsäureester
- Anlagerungsprodukte von Ethylenoxid an Fettsäurealkanolamide.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil® -Quat 3270 und 3272 (Hersteller: Th. Goldschmidt; diquatemäre Polydimethylsiloxane, Quaternium-80).

Alkylamidoamine, insbesondere Fettsäureamidoamine wie das unter der Bezeichnung Tego Amid® S 18 erhältliche Stearylamidopropyldimethylamin, zeichnen sich neben einer guten konditionierenden Wirkung speziell durch ihre gute biologische Abbaubarkeit aus. Ebenfalls sehr gut biologisch abbaubar sind quaternäre Esterverbindungen, sogenannte "Esterquats", wie die unter dem Warenzeichen Stepantex® vertriebenen Methyl-hydroxyalkyldialkoyloxyalkyl-ammoniummethosulfate sowie die unter dem Warenzeichen Dehyquart® vertriebenen Produkte wie Dehyquart® AU-46.

Ein Beispiel für ein als kationisches Tensid einsetzbares quaternäres Zuckerderivat stellt das Handelsprodukt Glucquat® 100 dar, gemäß INCI-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weiterhin können die erfindungsgemäßen Haarbehandlungsmittel bevorzugt noch einen konditionierenden Wirkstoff, ausgewählt aus der Gruppe, die von kationischen Tensiden, kationischen Polymeren, Alkylamidoaminen, Paraffinölen, pflanzlichen Ölen und synthetischen Ölen gebildet wird, enthalten.

Als konditionierende Wirkstoffe bevorzugt sein können kationische Polymere. Dies sind in der Regel Polymere, die ein quartäres Stickstoffatom, beispielsweise in Form einer Ammoniumgruppe, enthalten.

Bevorzugte kationische Polymere sind beispielsweise
- quatemisierte Cellulose-Derivate, wie sie unter den Bezeichnungen Celquat® und Polymer JR® im Handel erhältlich sind. Die Verbindungen Celquat® H 100, Celquat® L 200 und Polymer JR® 400 sind bevorzugte quaternierte Cellulose-Derivate.
- polymere Dimethyldiallylammoniumsalze und deren Copolymere mit Acrylsäure sowie mit Estern und Amiden von Acrylsäure und Methacrylsäure. Die unter den Bezeichnungen Merquat® 100 (Poly(dimethyldiallylammoniumchlorid)), Merquat® 550 (Dimethyldiallylammoniumchlorid-Acrylamid-Copolymer) und Merquat® 280 (Dimethyldiallylammoniumchlorid-Acrylsäure-Copolymer) im Handel erhältlichen Produkte sind Beispiele für solche kationischen Polymere.
- Copolymere des Vinylpyrrolidons mit quaternierten Derivaten des Dialkylaminoacrylats- und -methacrylats, wie beispielsweise mit Diethylsulfat quaternierte Vinylpyrrolidon-Dimethylaminomethacrylat-Copolymere. Solche Verbindungen sind unter den Bezeichnungen Gafquat® 734 und Gafquat® 755 im Handel erhältlich.
- Vinylpyrrolidon-Methoimidazoliniumchlorid-Copolymere, wie sie unter der Bezeichnung Luviquat® angeboten werden.
- quaternierter Polyvinylalkohol
sowie die unter den Bezeichnungen
- Polyquaternium 2,
- Polyquaternium 17,
- Polyquaternium 18 und
- Polyquaternium 27 bekannten Polymeren mit quartären Stickstoffatomen in der Polymerhauptkette.

Besonders bevorzugt sind kationische Polymere der vier erstgenannten Gruppen. Ganz besonders-bevorzugt sind Polyquaternium-2, Polyquaternium-10 und Polyquaternium-22.

Als konditionierende Wirkstoffe weiterhin geeignet sind Silikonöle, insbesondere Dialkyl- und Alkylarylsiloxane, wie beispielsweise Dimethylpolysiloxan und Methylphenylpolysiloxan, sowie deren alkoxylierte und quaternierte Analoga. Beispiele für solche Silikone sind die von Dow Corning unter den Bezeichnungen DC 190, DC 200, DC 344, DC 345 und DC 1401 vertriebenen Produkte sowie die Handelsprodukte Q2-7224 (Hersteller: Dow Corning; ein stabilisiertes Trimethylsilylamodimethicon), Dow Corning® 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil® -Quat 3270 und 3272 (Hersteller: Th. Goldschmidt; diquatemäre Polydimethylsiloxane, Quaternium-80).

Ebenfalls einsetzbar als konditionierende Wirkstoffe sind Paraffinöle, synthetisch hergestellte oligomere Alkene sowie pflanzliche Öle wie Jojobaöl, Sonnenblumenöl, Orangenöl, Mandelöl, Weizenkeimöl und Pfirsichkernöl.

Gleichfalls geeignete haarkonditionierende Verbindungen sind Phospholipide, beispielsweise Sojalecithin, Ei-Lecithin und Kephaline.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- zwitterionische und amphotere Polymere wie beispielsweise Acrylamidopropyl-trimethylammoniumchlorid/Acrylat-Copolymere und Octylacrylamid/Methyl-methacrylat/tert.Butylaminoethylmethacrylat/2- Hydroxypropylmethacrylat-Copolymere,
- anionische Polymere wie-beispielsweise Polyacrylsäuren, vemetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.Butyl-acrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Glucose und Maleinsäure,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsvermittler wie Ethanol, Isopropanol, Ethylenglykol, Propylenglykol, Glycerin und Diethylenglykol,
- Antischuppenwirkstoffe wie Piroctone Olamine und Zink Omadine,
- weitere Substanzen zur Einstellung des pH-Wertes,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien.

Die Bestandteile des wasserhaltigen Trägers werden zur Herstellung der erfindungsgemäßen Färbemittel in für diesen Zweck üblichen Mengen eingesetzt; z.B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

Die oxidative Entwicklung der Färbung kann grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Als Oxidationsmittel kommen Persulfate, Chlorite und insbesondere Wasserstoffperoxid oder dessen Anlagerungsprodukte an Harnstoff, Melamin sowie Natriumborat in Frage. Weiterhin ist es möglich, die Oxidation mit Hilfe von Enzymen durchzuführen. Dabei können die Enzyme zur Übertragung von Luftsauerstoff auf die Entwicklerkomponente oder zur Verstärkung der Wirkung geringer Mengen vorhandener Oxidationsmittel dienen. Ein Beispiel für ein enzymatisches Verfahren stellt das Vorgehen dar, die Wirkung geringer Mengen (z.B. 1 % und weniger, bezogen auf das gesamte Mittel) Wasserstoffperoxid durch Peroxidasen zu verstärken.

Zweckmäßigerweise wird die Zubereitung des Oxidationsmittels unmittelbar vor dem Haarefärben mit der Zubereitung aus den Oxidationsfarbstoffvorprodukten vermischt. Das dabei entstehende gebrauchsfertige Haarfärbepräparat sollte bevorzugt einen pH-Wert im Bereich von 6 bis 10 aufweisen. Besonders bevorzugt ist die Anwendung der Haarfärbemittel in einem schwach alkalischen Milieu. Die Anwendungstemperaturen können in einem Bereich zwischen 15 und 40 °C liegen. Nach einer Einwirkungszeit von ca. 30 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Die nachfolgenden Beispiele sollen den Erfindungsgegenstand näher erläutern.

### Ausführungsbeispiele

Die nachfolgend aufgeführten Farbstoffkombinationen wurden in eine Rezeptur der folgenden Zusammensetzung eingearbeitet:

| Rohstoff | Menge |
|---|---|
| Fettalkoholgemisch C₁₂-C₁₈ | 5,25 Gew.-% |
| Eumulgin® B2¹ | 0,4 Gew.-% |
| Texapon® NSO² | 10,0 Gew.-% |
| Dehyton® K³ | 6,25 Gew.-% |
| Natriumsulfit | 0,3 Gew.-% |
| Ammoniumsulfat | 0,5 Gew.-% |
| Natrosol® 250 HR⁴ | 1,0 Gew.-% |
| Entwicklerkomponente | 0,1 mol/l |
| Kupplerkomponente | 0,1 mol/l |
| Ammoniak, 25%ig | ad pH 6 beziehungsweise 9 |
| Wasser | ad 100 Gew.-% |

| | |
|---|---|
| ¹ Cetylstearylalkohol mit ca. 20 EO-Einheiten (INCI-Bezeichnung: Ceteareth-20) (Henkel) | |
| ² Natriumsalz des Laurylethersulfats (ca. 27% Aktivsubstanz; INCI-Bezeichnung: Sodium Laureth Sulfate) (Henkel) | |
| ³ Fettsäureamid mit Betainstruktur (ca. 30% Aktivsubstanz; INCI-Bezeichnung: Cocamidopropyl Betaine) (Henkel) | |
| ⁴ Hydroxyethylcellulose (Hercules) | |

Nach Einstellung des jeweiligen pH-Wertes wurde die Färbelösung mit einer kommerziellen 6%igen Wasserstoffperoxidzubereitung (Marktprodukt Poly Color Brillance) im Verhältnis 1:1 verdünnt. Die Anwendungszubereitungen wurden auf Haarsträhnen (Kerling, naturweiß) aufgetragen. Nach 30min Einwirkzeit bei 32°C wurden die Haarsträhnen gespült, getrocknet und das erhaltene Färberesultat visuell nach dem Taschenlexikon der Farben (2. Auflage, Musterschmidt-Verlag Zürich-Göttingen, 1975) bestimmt. Es wurden die folgenden Färbungen mit 4-Amino-2-((diethylamino)-methyl)phenol als Entwicklerkomponente durchgeführt:

| Kupplerkomponente | Ausfärbung bei pH 6 | Ausfärbung bei pH 9 |
|---|---|---|
| --- | Nicht bestimmt | Kakaobraun |
| 2,4-Diaminophenoxyethanol * | Braungrau | Violettbraun |
| 1,3-Bis-(2,4-diamino-phenoxy)-propan * | Graubraun | Violettbraun |
| 6-Methyl-1,2,3,4-tetrahydrochinoxalin * | Hellgelb | Hellgelb |
| 3-Amino-2-chlor-6-methylphenol | Nicht bestimmt | Rotbraun |
| 2,4-Dichlor-3-aminophenol | Blaßgelb | Dunkelbraun |
| 1,5-Dihydroxynaphthalin * | Gelbweiß | Madeirabraun |
| 2,7-Dihydroxynaphthalin * | Gelbweiß | Bambusgelb |
| 3-Trifluoracetylamino-2-chlor-6-methylphenol | Gelbweiß | Rotbraun |
| 2-Amino-5-chlor-3-hydroxypyridin * | Mattgelb | Englischrot |
| 5-Amino-4-chlor-2-methylphenol | Blaßorange | Braunrot |
| 4-Hydroxyindol * | Sandfarbig | Rotbraun |
| 6-Hydroxyindol * | Mattgelb | Haarbraun |
| 3,4-Diaminobenzoesäure * | Gelbbraun | Gelbweiß |
| 1-Phenyl-3-methylpyrazol-5-on * | Gelbweiß | Mattrot |
| 3,5-Diamino-2,6-dimethoxypyridin * | Dunkelgrün | Dunkelgrün |
| 2,6-Bis-(2'hydroxyethylamino)-1-methylbenzol * | Graurosa | Tiefmagenta |

| | | |
|---|---|---|
| * nicht erfindungsgemäß | | |

## Patentansprüche

1. Mittel zum Färben keratinischer Fasern, **dadurch gekennzeichnet, daß** es
(a) mindestens eine Entwicklerkomponente der Formel (I) 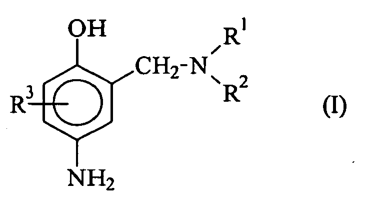 wobei R¹ und R² unabhängig voneinander für eine C₁- bis C₄-Alkylgruppe stehen und R³ für Wasserstoff, ein Halogenatom, eine C₁- bis C₄-Alkylgruppe, eine C₁- bis C₄-Alkoxygruppe oder eine C₁- bis C₄-Hydroxyalkylgruppe steht
und
(b) mindestens eine Kupplerkomponente, ausgewählt aus einer Gruppe, gebildet von 3-Amino-2-chlor-6-methylphenol, 3-Trifluoroacetylamino-2-chlor-6-methylphenol, 5-Amino-4-chlor-2-methylphenol, 2,4-Dichlor-3-aminophenol enthält.

2. Mittel nach Anspruch 1, **dadurch gekennzeichnet, daß** der Rest R³ der Verbindung der Formel (I) für Wasserstoff steht.

3. Mittel nach Anspruch 2, **dadurch gekennzeichnet, daß** die Verbindung der Formel (I) 4-Amino-2-((diethylamino)methyl)phenol ist.

4. Oxidationsfärbemittel nach einem der Ansprüche 1 bis 3, **dadurch gekennzeichnet, daß** es weiterhin mindestens eine Entwicklerkomponente, ausgewählt aus der Gruppe, die aus p-Phenylendiamin, p-Toluylendiamin, p-Aminophenol, 1-(2'-Hydroxyethyl)-2,5-diaminobenzol, 4-Amino-3-methylphenol, 2,4,5,6-Tetraaminopyrimidin, 2-Hydroxy-4,5,6-triaminopyrimidin, 4-Hydroxy-2,5,6-triaminopyrimidin, 4,5-Diamino-1-(2-hydroxyethyl)-pyrazol, 2-Aminomethyl-4-amino-phenol, N,N-Bis-(2-hydroxyethyl)-p-phenylendiamin, Bis-(2-hydroxy-5-aminophenyl)-methan, 1, 10-Bis-(2,5-diaminophenyl) -1,4,7,10-tetraoxadecan und o-Aminophenol besteht, enthält.

5. Oxidationsfärbemittel nach einem der Ansprüche 1 bis 4, **dadurch gekennzeichnet, daß** es mindestens eine weitere Kupplerkomponente, ausgewählt aus der Gruppe, die gebildet wird von 1-Naphthol, 5-Amino-2-methylphenol, 2-Amino-3-hydroxypyridin, 3-Amino-2-methylamino-6-methoxypyridin, Resorcin, 4-Chlorresorcin, 2-Methylresorcin, m-Aminophenol und o-Aminophenol, enthält.

6. Oxidationsfärbemittel nach einem der Ansprüche 1 bis 5, **dadurch gekennzeichnet, daß** Entwicklerkomponenten in einer Menge von 0,005 bis 20 Gew.-%, vorzugsweise 0,1 bis 5 Gew.-%, und Kupplerkomponenten in einer Menge von 0,005 bis 20 Gew.-%, vorzugsweise 0,1 bis 5 Gew.-%, jeweils bezogen auf das gesamte Oxidationsfärbemittel, enthalten sind.

7. Oxidationsfärbemittel nach einem der Ansprüche 1 bis 6, **dadurch gekennzeichnet, daß** mindestens ein direktziehender Farbstoff enthalten ist.

8. Oxidationsfärbemittel nach einem der Ansprüche 1 bis 7, **dadurch gekennzeichnet, daß** mindestens ein Indol- und/oder mindestens ein Indolinderivat enthalten ist.

9. Verwendung von Mitteln nach einem der Ansprüche 1 bis 8 zum Färben keratinischer Fasern.

## Claims

1. Agent for colouring keratin fibres, **characterized in that** it comprises
(a) at least one developer component of the formula (I) 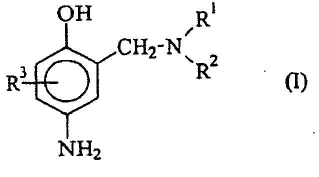 where R¹ and R², independently of one another, are a C₁- to C₄-alkyl group and R³ is hydrogen, a halogen atom, a C₁- to C₄-alkyl group, a C₁- to C₄-alkoxy group or a C₁- to C₄-hydroxyalkyl group and
(b) at least one coupler component chosen from the group formed by 3-amino-2-chloro-6-methylphenol, 3-trifluoroacetylamino-2-chloro-6-methylphenol, 5-amino-4-chloro-2-methylphenol, 2,4-dichloro-3-aminophenol.

2. Agent according to Claim 1, **characterized in that** the radical R³ of the compound of the formula (I) is hydrogen.

3. Agent according to Claim 2, **characterized in that** the compound of the formula (I) is 4-amino-2-((diethylamino)methyl)phenol.

4. Oxidation colorant according to one of Claims 1 to 3, **characterized in that** it further comprises at least one developer component chosen from the group which consists of p-phenylenediamine, p-tolylenediamine, p-aminophenol, 1-(2'-hydroxyethyl)-2,5-diaminobenzene, 4-amino-3-methylphenol, 2,4,5,6-tetraaminopyrimidine, 2-hydroxy-4,5,6-triaminopyrimidine, 4-hydroxy-2,5,6-triaminopyrimidine, 4,5-diamino-1-(2-hydroxyethyl)-pyrazole, 2-aminomethyl-4-aminophenol, N,N-bis(2-hydroxyethyl)-p-phenylenediamine, bis(2-hydroxy-5-aminophenyl)methane, 1,10-bis(2,5-diaminophenyl)-1,4,7,10-tetraoxadecane and o-aminophenol.

5. Oxidation colorant according to one of Claims 1 to 4, **characterized in that** it comprises at least one further coupler component chosen from the group which is formed by 1-naphthol, 5-amino-2-methylphenol, 2-amino-3-hydroxypyridine, 3-amino-2-methylamino-6-methoxypyridine, resorcinol, 4-chlororesorcinol, 2-methylresorcinol, m-aminophenol and o-aminophenol.

6. Oxidation colorant according to one of Claims 1 to 5, **characterized in that** developer components are present in an amount of from 0.005 to 20% by weight, preferably 0.1 to 5% by weight, and coupler components are present in an amount of from 0.005 to 20% by weight, preferably 0.1 to 5% by weight, in each case based on the total oxidation colorant.

7. Oxidation colorant according to one of Claims 1 to 6, **characterized in that** at least one direct dye is present.

8. Oxidation colorant according to one of Claims 1 to 7, **characterized in that** at least one indole derivative and/or at least one indoline derivative is present.

9. Use of agents according to one of Claims 1 to 8 for colouring keratin fibres.

## Revendications

1. Agent pour teindre les fibres kératiniques **caractérisé en ce qu'**il contient,
(a) au moins un composant révélateur de formule (I) 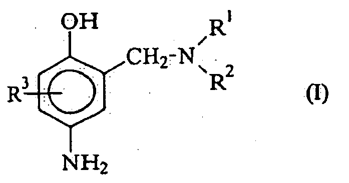 dans laquelle R¹ et R² représentent indépendamment l'un de l'autre un groupe alkyle en C₁ à C₄ et R³ représente un atome d'hydrogène, un atome d'halogène, un groupe alkyle en C₁ à C₄, un groupe alcoxy en C₁ à C₄ ou un groupe hydroxyalkyle en C₁ à C₄
et
(b) au moins un composant de couplage, choisi dans le groupe formé par le 3-amino-2-chloro-6-méthylphénol, le 3-trifluoroacétylamino-2-chloro-6-méthylphénol, le 5-amino-4-chloro-2-méthylphénol, le 2,4-dichloro-3-aminophénol.

2. Agent selon la revendication 1, **caractérisé en ce que** le radical R³ du composé de formule (I) représente un atome d'hydrogène.

3. Agent selon la revendication 2, **caractérisé en ce que** le composé de formule (I) est le 4-amino-2-((diéthylamino)méthyl)phénol.

4. Teinture par oxydation selon l'une quelconque des revendications 1 à 3, **caractérisé en ce qu'**elle contient en outre au moins un composant révélateur, choisi dans le groupe qui est constitué par la p-phénylènediamine, la p-toluylènediamine, le p-aminophénol, le 1-(2'-hydroxyéthyl)-2,5-diaminobenzène, le 4-amino-3-méthylphénol, la 2,4,5,6-tétraaminopyrimidine, la 2-hydroxy-4,5,6-triaminopyrimidine, la 4-hydroxy-2,5,6-triaminopyrimidine, le 4,5-diamino-1-(2-hydroxyéthyl)-pyrazole, le 2-aminométhyl-4-aminophénol, la N,N-bis-(2-hydroxyéthyl)-p-phénylènediamine, le bis-(2-hydroxy-5-aminophényl)-méthane, le 1,10-bis-(2,5-diaminophényl)-1,4,7,10-tétraoxadécane et l'o-amino-phénol.

5. Teinture par oxydation selon l'une quelconque des revendications 1 à 4, **caractérisé en ce qu'**elle contient au moins un composant de couplage supplémentaire, choisi dans le groupe qui est formé par le 1-naphtol, le 5-amino-2-méthylphénol, la 2-amino-3-hydroxypyridine, la 3-amino-2-méthylamino-6-méthoxypyridine, la résorcine, la 4-chlororésorcine, la 2-méthylrésorcine, le m-aminophénol et l'o-aminophénol.

6. Teinture par oxydation selon l'une quelconque des revendications 1 à 5, **caractérisé en ce que** des composants révélateurs sont contenus en une quantité de 0,005 à 20% en poids, de préférence de 0,1 à 5% en poids, et des composants de couplage en une quantité de 0,005 à 20% en poids, de préférence de 0,1 à 5% en poids, à chaque fois par rapport à la teinture par oxydation totale.

7. Teinture par oxydation selon l'une quelconque des revendications 1 à 6, **caractérisé en qu'**au moins un colorant montant directement sur les fibres est contenu.

8. Teinture par oxydation selon l'une quelconque des revendications 1 à 7, **caractérisé en ce qu'**au moins un dérivé indole et/ou au moins un dérivé indoline sont contenus.

9. Utilisation d'agents selon l'une quelconque des revendications 1 à 8 pour teindre les fibres kératiniques.
